(19) Europäisches Patentamt

European Patent Office

Office européen des brevets

(11) Veröffentlichungsnummer: **0 534 125 B1**

(12) **EUROPÄISCHE PATENTSCHRIFT**

(45) Veröffentlichungstag der Patentschrift: **05.07.95**

(51) Int. Cl.6: **C07C 7/08**

(21) Anmeldenummer: **92114029.9**

(22) Anmeldetag: **18.08.92**

(54) **Verfahren zur Aufarbeitung des Sumpfproduktes einer Extraktivdestillation zur Gewinnung reiner Kohlenwasserstoffe.**

(30) Priorität: **25.09.91 DE 4131938**

(43) Veröffentlichungstag der Anmeldung:
**31.03.93 Patentblatt 93/13**

(45) Bekanntmachung des Hinweises auf die Patenterteilung:
**05.07.95 Patentblatt 95/27**

(84) Benannte Vertragsstaaten:
**BE DE FR GB IT NL**

(56) Entgegenhaltungen:
**EP-A- 0 046 876**
**EP-A- 0 065 194**

(73) Patentinhaber: **Krupp Koppers GmbH**
**Altendorfer Strasse 120**
**D-45143 Essen (DE)**

(72) Erfinder: **Kaiping, Martin**
**Curtiusstrasse 76**
**W-4300 Essen 1 (DE)**
Erfinder: **Klaumünzner, Udo**
**Ursulastrasse 18**
**W-4330 Mülheim/Ruhr (DE)**
Erfinder: **Schneider, Hans-Christoph**
**Rosental 8**
**W-4320 Hattingen/Ruhr (DE)**
Erfinder: **Vollmer, Hans-Jürgen, Dr.**
**Dahlienhof 13**
**W-4300 Essen 1 (DE)**

**Beschreibung**

Die Erfindung betrifft ein Verfahren zur Aufarbeitung des Sumpfproduktes einer Extraktivdestillation zur Gewinnung reiner Kohlenwasserstoffe unter Verwendung von N-substituierten Morpholinen, deren Substituenten nicht mehr als 7 C-Atome enthalten, als Lösungsmittel, bei dem das aufzuarbeitende Sumpfprodukt aus der Extraktivdestillationskolonne in den mittleren Teil einer mit einem Sumpfkocher versehenen Abtriebskolonne eingeleitet wird, in der die zu gewinnenden Kohlenwasserstoffe über Kopf abdestilliert werden, während das Lösungsmittel aus dem Sumpf der Abtriebskolonne abgezogen und nach entsprechender Abkühlung zur Lösungsmittelaufgabe der Extraktivdestillationskolonne zurückgeführt wird.

Die Extraktivdestillation kohlenwasserstoffhaltiger Einsatzprodukte mit den vorstehend genannten N-substituierten Morpholinen, insbesondere mit N-Formylmorpholin, als selektivem Lösungsmittel ist bereits seit längerer Zeit bekannt und wird heute im großtechnischen Maßstab vor allem zur Gewinnung hochreiner Aromaten eingesetzt. Das Verfahren ist aber auch zur Gewinnung anderer Stoffklassen, wie z.B. der Olefine und Diolefine, geeignet. Die aus den entsprechenden Einsatzprodukten zu gewinnenden Kohlenwasserstoffe reichern sich dabei zusammen mit der Hauptmenge des Lösungsmittels im Sumpf der Extraktivdestillationskolonne an und müssen in einer nachgeschalteten Abtriebskolonne vom Lösungsmittel abgetrennt werden. Hierbei werden die zu gewinnenden Kohlenwasserstoffe über Kopf aus der Abtriebskolonne abdestilliert, während das Lösungsmittel als Sumpfprodukt dieser Kolonne anfällt und von dort seiner Wiederverwendung zugeführt werden kann. Hierbei kann die Abtriebskolonne zu ihrer Beheizung neben den üblichen Sumpfkochern noch Seitenkocher aufweisen, die in erster Linie den auf die Abtriebskolonne aufgegebenen Rückfluß wieder verdampfen sollen.

Bei der Durchführung des vorstehend beschriebenen Verfahrens ist man selbstverständlich bestrebt, in der Abtriebskolonne eine möglichst vollständige Abtrennung der zu gewinnenden Kohlenwasserstoffe vom Lösungsmittel zu erzielen, um auf diese Weise die Verluste an zu gewinnenden Kohlenwasserstoffen möglichst niedrig zu halten. Bei der bisher üblichen Arbeitsweise konnte dies jedoch nur dadurch erreicht werden, daß im Sumpf der Abtriebskolonne mit einer so hohen Betriebstemperatur gearbeitet wurde, daß zur Kolonnenbeheizung Hochdruckdampf erforderlich war, während zur Beheizung der Extraktivdestillationskolonne normalerweise der wesentlich kostengünstigere Mittel- bzw. Niederdruckdampf ausreicht. Ein weiterer Nachteil des Arbeitens mit verhältnismäßig hohen Temperaturen in der Abtriebskolonne ist in der damit verbundenen hohen thermischen Belastung des Lösungsmittels zu sehen, die natürlich die thermische Zersetzung des Lösungsmittels begünstigt.

Der Erfindung liegt deshalb die Aufgabe zugrunde, das Verfahren zum Abtrieb der zu gewinnenden Kohlenwasserstoffe aus dem Sumpfprodukt der gattungsgemäßen Extraktivdestillation dahingehend zu verbessern, daß ein gutes Trennergebnis bei einer möglichst niedrigen Betriebstemperatur im Sumpf der Abtriebskolonne erzielt werden kann, so daß für die Beheizung dieser Kolonne Niederdruckdampf verwendet werden kann und gleichzeitig eine hohe thermische Belastung des Lösungsmittels vermieden wird.

Diese Aufgabe wird gelöst durch ein Verfahren der eingangs genannten Art, das erfindungsgemäß dadurch gekennzeichnet ist, daß das aus dem Sumpf der Abtriebskolonne abgezogene Lösungsmittel vor der Wiedereinleitung in die Extraktivdestillationskolonne in einen Verdampfer eingeleitet wird, der mit einem Druck $p_2$ betrieben wird, der niedriger ist als der Druck $p_1$ im Sumpf der Abtriebskolonne, wobei die aus dem Verdampfer austretenden Dämpfe kondensiert, gesammelt und in die Abtriebskolonne zurückgeführt werden, während das Lösungsmittel im flüssigen Zustande aus dem Sumpf des Verdampfers abgezogen und in die Extraktivdestillationskolonne wiedereingeleitet wird.

Das heißt, beim erfindungsgemäßen Verfahren wird das aus dem Sumpf der Abtriebskolonne abgezogene Lösungsmittel einer nochmaligen Teilverdampfung bei niedrigerem Druck als dem im Sumpf der Abtriebskolonne herrschenden Druck unterzogen, was einen höheren Abtrieb der zu gewinnenden Kohlenwasserstoffe aus dem Lösungsmittel erlaubt. Da das Lösungsmittel somit mit höherer Reinheit in die Extraktivdestillationskolonne zurückgeführt werden kann, bewirkt dies eine Verbesserung des Trennergebnisses der Extraktivdestillation. Gleichzeitig ist durch den geringen Druck im Verdampfer eine Senkung der Sumpftemperatur der Abtriebskolonne möglich, so daß diese mit preiswertem Niederdruckdampf betrieben werden kann. Dies bedeutet neben dem erzielbaren Kostenvorteil auch eine geringere thermische Belastung des Lösungsmittels. Vorzugsweise werden hierbei die Abtriebskolonne und der Verdampfer mit gleicher Temperatur betrieben.

In welchem Umfang bei der Durchführung des erfindungsgemäßen Verfahrens der Druck $p_2$ im Verdampfer gegenüber dem Druck $p_1$ im Sumpf der Abtriebskolonne abgesenkt werden muß, hängt natürlich von den durch das Verfahren zu gewinnenden Kohlenwasserstoffen sowie vom verwendeten Lösungsmittel ab. Im konkreten Anwendungsfall müssen hierbei die Verfahrensparameter so variiert werden, daß das gewünschte Optimum, das heißt weitestgehende Reduzierung des Kohlenwasserstoffgehaltes im abgetriebenen Lösungsmittel bei gleichzeitiger Senkung der Sumpftemperatur in der Abtriebskolonne, gefunden wird. Generell ist davon auszugeen, daß der Wert für $p_2$ frei wählbar ist, wobei $p_2$ natürlich niedriger als $p_1$ sein muß. Bei der Gewinnung von Reinbenzol unter Verwendung von N-Formylmorpholin (NFM) als Lösungsmittel kann beispielsweise $p_2$ zwischen 0,15 und 0,077 bar betragen, während der Wert für $p_1$ in diesem Falle bei 0,368 bar liegt.

Für die Durchführung des erfindungsgemäßen Verfahrens werden zwar zusätzlich ein Verdampfer, ein Kühler, ein Sammelbehälter und eine Pumpe benötigt. Dieser zusätzliche apparative Mehraufwand wird jedoch dadurch weitgehend kompensiert, daß an der Abtriebskolonne ein zweiter Sumpfkocher sowie in der Regel Seitenkocher entfallen können.

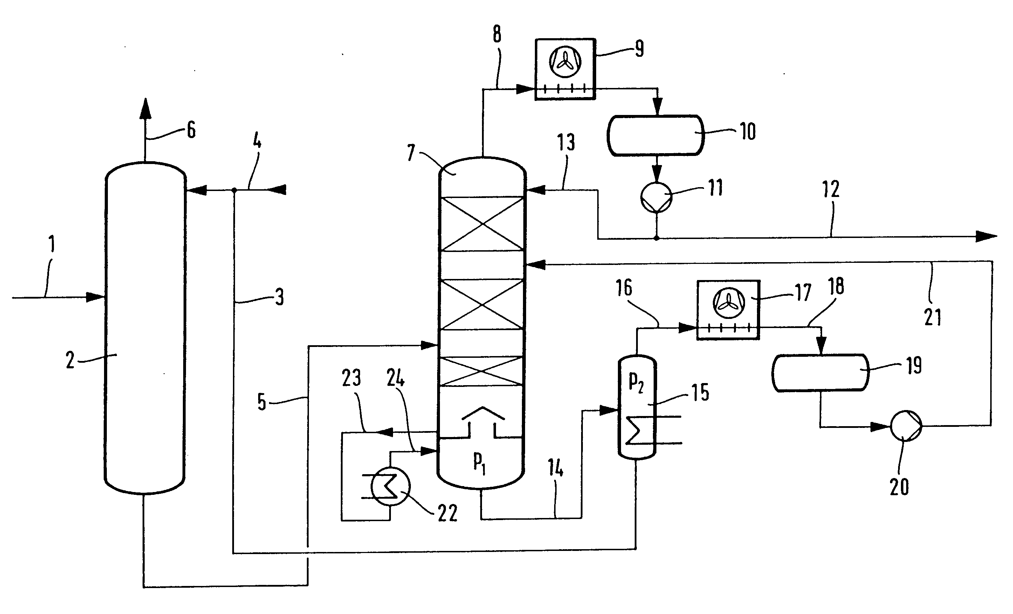

Nachfolgend soll das erfindungsgemäße Verfahren an Hand des in der Abbildung dargestellten Fließschemas weiter erläutert werden. Darin sind selbstverständlich nur die zur Erläuterung des Verfahrensablaufes unbedingt notwendigen Anlagenteile dargestellt, während sonstige Nebeneinrichtungen, wie Ventile, Wärmetauscher, Meß- und Regeleinrichtungen sowie sonstige Nebeneinrichtungen, nicht wiedergegeben werden.

Das aufzuarbeitende Einsatzprodukt wird hierbei über die Leitung 1 in den mittleren Teil der Extraktivdestillationskolonne 2 eingeleitet. Da die Extraktivdestillation nicht Gegenstand der vorliegenden Erfindung ist, braucht auf Einzelheiten dieser Verfahrensstufe nicht näher eingegangen zu werden. Bei der Extraktivdestillationskolonne 2 kann es sich um eine Kolonne üblicher Bauart handeln, die mit Böden oder sonstigen Einbauten versehen ist. Gleichzeitig mit dem Einsatzprodukt wird der Extraktivdestillationskolonne 2 das erforderliche Lösungsmittel über die Leitung 3 am Kopf zugeführt. Die Leitung 4 dient der Zufuhr von frischem Lösungsmittel. In der Extraktivdestillationskolonne 2 reichern sich die aus dem Einsatzprodukt abzutrennenden Kohlenwasserstoffe zusammen mit dem Lösungsmittel im Sumpf dieser Kolonne an, wobei das anfallende Sumpfprodukt aus dieser Kolonne über die Leitung 5 abgezogen wird. Gleichzeitig entweichen die leichter siedenden Bestandteile des Einsatzproduktes über Kopf aus der Extraktivdestillationskolonne 2 und werden über die Leitung 6 ihrer weiteren Behandlung zugeführt. Das Sumpfprodukt der Extraktivdestillation gelangt über die Leitung 5 zur Abtriebskolonne 7 und wird in den mittleren Teil dieser mit Böden oder sonstigen Einbauten versehenen Kolonne eingeleitet. In der Abtriebskolonne 7 werden die zu gewinnenden reinen Kohlenwasserstoffe destillativ vom Lösungsmittel abgetrennt. Diese Kohlenwasserstoffe werden daher dampfförmig über Kopf aus der Abtriebskolonne 7 abgezogen und gelangen über die Leitung 8 in den Kühler 9, in dem sie kondensiert werden, um anschließend in den Sammelbehälter 10 zu gelangen. Aus dem Sammelbehälter 10 werden die reinen Kohlenwasserstoffe im flüssigen Zustand mittels der Pumpe 11 abgezogen. Die Hauptmenge wird dabei über die Leitung 12 ihrer weiteren Verwendung bzw. Verarbeitung zugeführt, während ein kleiner Teilstrom über die Leitung 13 als Rückfluß auf die Abtriebskolonne 7 wiederaufgegeben wird. Das von den zu gewinnenden Kohlenwasserstoffen weitgehend befreite Lösungsmittel kann währenddessen über die Leitung 14 aus dem Sumpf der Abtriebskolonne 7 abgezogen werden. Während bei der bisher üblichen Arbeitsweise das Lösungsmittel nach entsprechender Abkühlung unmittelbar zur Extraktivdestillationskolonne zurückgeführt wurde, gelangt es erfindungsgemäß zunächst in den Verdampfer 15, in dem es eine weitere Teilverdampfung erfährt. Wie bereits gesagt wurde, ist hierbei der Druck $p_2$ im Verdampfer 15 niedriger als der Druck $p_1$ im Sumpf der Abtriebskolonne 7. Die aus dem Verdampfer 15 austretenden Dämpfe, bei denen es sich im wesentlichen um aus dem Lösungsmittel noch abgetriebene Kohlenwasserstoffe handelt, werden über die Leitung 16 abgezogen und gelangen nach Kondensation im Kühler 17 über die Leitung 18 in den Sammelbehälter 19. Von dort werden die Kohlenwasserstoffe im flüssigen Zustand durch die Pumpe 20 über die Leitung 21 zur Abtriebskolonne 7 zurückgeführt. Die Wiedereinleitung in diese Kolonne erfolgt hierbei vorzugsweise zwischen der Aufgabestelle für das Sumpfprodukt aus der Extraktivdestillation und der Aufgabestelle für den Rückfluß. Das Lösungsmittel, das nunmehr eine sehr hohe Reinheit aufweist, wird im flüssigen Zustand aus dem Sumpf des Verdampfers 15 abgezogen und über die Leitung 3 in die Extraktivdestillationskolonne 2 wiedereingeleitet. Hierbei erfährt das Lösungsmittel vor seiner Wiedereinleitung die notwendige Abkühlung, wobei dies in an sich bekannter Weise im indirekten Wärmeaustausch mit anderen Produktströmen des Verfahrens erfolgen kann. Einzelheiten dieser Abkühlung sind im Fließschema nicht dargestellt, da dies nicht Gegenstand der vorliegenden Erfindung ist. Am Fuße der Abtriebskolonne 7 ist der Sumpfkocher 22 angeordnet, der über die Leitungen 23 und 24 mit der Abtriebskolonne 7 verbunden ist, und der in diesem Falle für die notwendige Wärmezufuhr zu dieser Kolonne ausreicht. Bei dem beim erfindungsgemäßen Verfahren zur Anwendung gelangenden relativ niedrigen Temperaturniveau kann für die Beheizung des Sumpfkochers 22 Niederdruckdampf verwendet werden. Weitere Sumpf- bzw. Seitenkocher sind normalerweise in diesem Falle für den Betrieb der Abtriebskolonne 7 nicht erforderlich. Wegen des relativ niedrigen Temperaturniveaus in der Abtriebskolonne 7 ist es gleichzeitig möglich, die Kopftemperatur in dieser Kolonne so niedrig einzustellen, daß für den Kühler 9 ein Luftkühler verwendet werden kann.

3

Die Wirksamkeit des erfindungsgemäßen Verfahrens wird durch das nachfolgende Anwendungsbeispiel belegt. Hierbei ging es um die Gewinnung von Reinbenzol durch Extraktivdestillation mit N-Formylmorpholin (NFM) als Lösungsmittel, was einen wesentlichen Anwendungsfall des erfindungsgemäßen Verfahrens darstellt. Das aus der Extraktivdestillationskolonne 2 abgezogene Sumpfprodukt hatte dabei folgende Zusammensetzung:

| Benzol | 20 956 kg/h |
|---|---|
| NFM | 90 946 kg/h |
| Methylcyclohexan | 3,25 kg/h |
| Dimethylcyclopentan | 1,997 kg/h |

Zur Abtrennung des Benzols vom Lösungsmittel (NFM) wurde dieses Sumpfprodukt in die Abtriebskolonne eingeleitet. Die bei der bisher üblichen Arbeitsweise ohne zusätzlichen Verdampfer erzielten Ergebnisse sind in der nachfolgenden Tabelle 1 zusammengefaßt.

## Tabelle 1

| | Temp. | Druck | Benzol-Gehalt im NFM |
|---|---|---|---|
| Sumpf Abtriebskolonne (bisherige Schaltung) | °C | bar | % |
| | ca. 195 | ca. 0,55 | ca. 1 |
| | ca. 185 | ca. 0,37 | ca. 0,55 |

Bei Anwendung des erfindungsgemäßen Verfahrens ließen sich dagegen bei verschiedenen Temperaturen und unterschiedlichen Drücken im Verdampfer die in der nachfolgenden Tabelle 2 zusammengefaßten Ergebnisse erzielen:

Tabelle 2

| | Temp. | Druck | Benzol-Gehalt im NFM |
|---|---|---|---|
| | °C | bar | % |
| Sumpf Abtriebskolonne vor Verdampfer | 125 | 0,368 | 4,2 |
| NFM zur ED, nach Verdampfer | 125 125 | 0,15 0,077 | 1,4 0,5 |
| Sumpf Abtriebskolonne vor Verdampfer | 140 | 0,368 | 2,8 |
| NFM zur ED nach Verdampfer | 140 140 | 0,15 0,116 | 0,8 0,5 |

Die vorliegenden Ergebnisse lassen erkennen, daß mit dem zusätzlichen Verdampfer, der auf einem niedrigeren Druckniveau als die Abtriebskolonne arbeitet, ein weiterer Benzolabtrieb aus dem Lösungsmittel möglich ist, wobei ein Benzolgehalt von 0,5 % im Lösungsmittel erreicht werden kann. Ein so geringer Benzolgehalt konnte bei dem bisher üblichen Verfahren nur bei Anwendung hoher Temperaturen und niedriger Drücke erzielt werden. Die beim erfindungsgemäßen Verfahren auftretenden relativ hohen Benzolgehalte im Sumpfprodukt der Abtriebskolonne sind durch die niedrigen Betriebstemperaturen dieser Kolonne bedingt. Diese niedrigen Temperaturen gestatten jedoch die Beheizung der Abtriebskolonne mit

EP 0 534 125 B1

kostengünstigem Niederdruckdampf, wobei die relativ hohen Benzolgehalte im Sumpfprodukt der Abtriebskolonne wegen der nachfolgenden Weiterbehandlung desselben im Verdampfer nicht stören.

**Patentansprüche**

1. Verfahren zur Aufarbeitung des Sumpfproduktes einer Extraktivdestillation zur Gewinnung reiner Kohlenwasserstoffe unter Verwendung von N-substituierten Morpholinen, deren Substituenten nicht mehr als 7 C-Atome enthalten, als Lösungsmittel, bei dem das aufzuarbeitende Sumpfprodukt aus der Extraktivdestillationskolonne in den mittleren Teil einer mit einem Sumpfkocher versehenen Abtriebskolonne eingeleitet wird, in der die zu gewinnenden Kohlenwasserstoffe über Kopf abdestilliert werden, während das Lösungsmittel aus dem Sumpf der Abtriebskolonne abgezogen und nach entsprechender Abkühlung zur Lösungsmittelaufgabe der Extraktivdestillationskolonne zurückgeführt wird, **dadurch gekennzeichnet**, daß das aus dem Sumpf der Abtriebskolonne abgezogene Lösungsmittel vor der Wiedereinleitung in die Extraktivdestillationskolonne in einen Verdampfer eingeleitet wird, der mit einem Druck $p_2$ betrieben wird, der niedriger ist als der Druck $p_1$ im Sumpf der Abtriebskolonne, wobei die aus dem Verdampfer austretenden Dämpfe kondensiert, gesammelt und in die Abtriebskolonne zurückgeführt werden, während das Lösungsmittel im flüssigen Zustand aus dem Sumpf des Verdampfers abgezogen und in die Extraktivdestillationskolonne wiedereingeleitet wird.

2. Verfahren nach Anspruch 1, **dadurch gekennzeichnet**, daß die Abtriebskolonne und der Verdampfer mit gleicher Temperatur betrieben werden.

3. Verfahren nach den Ansprüchen 1 bis 2, **dadurch gekennzeichnet**, daß die kondensierten Dämpfe aus dem Verdampfer zwischen der Aufgabestelle für das Sumpfprodukt aus der Extraktivdestillation und der Aufgabestelle für den Rückfluß in die Abtriebskolonne wiedereingeleitet werden.

**Claims**

1. Process for the work-up of the bottom product of an extractive distillation for obtaining pure hydrocarbons with the use, as solvent, of N-substituted morpholines, the substituents of which do not contain more than 7 C atoms, in which process the bottom product to be worked up is passed from the extractive distillation column into the central section of a column stripper furnished with a bottoms reboiler, in which column stripper the hydrocarbons to be obtained are distilled off via the head of the column, whereas the solvent is withdrawn from the bottom of the column stripper and, following appropriate cooling, is returned to the solvent delivery of the extractive distillation column, **characterised in that** the solvent withdrawn from the bottom of the column stripper, prior to reintroduction into the extractive distillation column, is introduced into an evaporator, which is operated at a pressure $p_2$, which is lower than the pressure $p_1$ in the bottom of the column stripper, the vapours leaving the evaporator being condensed, collected and returned to the column stripper, whereas the solvent is withdrawn in the liquid state from the bottom of the evaporator and reintroduced into the extractive distillation column.

2. Process according to Claim 1, **characterised in that** the column stripper and the evaporator are operated at the same temperature.

3. Process according to Claims 1 to 2, **characterised in that** the condensed vapours from the evaporator are reintroduced into the column stripper between the delivery point for the bottom product from the extractive distillation and the delivery point for the reflux.

**Revendications**

1. Procédé de retraitement du produit de bas de colonne d'une distillation extractive en vue de l'extraction d'hydrocarbures purs par utilisation de morpholines N-substituées, dont les substituants ne contiennent pas plus de 7 atomes de C, en tant que solvant, lors duquel le produit de bas de colonne à retraiter en provenance de la colonne de distillation extractive est introduit dans la partie médiane d'une colonne de rectification pourvue d'un rebouilleur de bas de colonne, dans laquelle les hydrocarbures à extraire sont éliminés par distillation en tête de colonne, alors que le solvant est retiré du bas de la colonne de rectification et est reconduit, après un refroidissement correspondant, à l'alimentation en solvant de la

5

colonne de distillation extractive, **caractérisé en ce que** le solvant retiré du bas de la colonne de rectification est introduit, avant la réintroduction dans la colonne de distillation extractive, dans un évaporateur, qui fonctionne avec une pression $p_2$, qui est inférieure à la pression $p_1$ dans le bas de la colonne de rectification, les vapeurs sortant de l'évaporateur étant condensées, recueillies et reconduites dans la colonne de rectification, alors que le solvant à l'état liquide est retiré du bas de l'évaporateur et est réintroduit dans la colonne de distillation extractive.

2. Procédé selon la revendication 1, **caractérisé en ce que** la colonne de rectification et l'évaporateur opèrent à la même température.

3. Procédé selon les revendications 1 à 2, **caractérisé en ce que** les vapeurs condensées en provenance de l'évaporateur sont réintroduites entre le point d'alimentation pour le produit de bas de colonne en provenance de la distillation extractive et le point d'alimentation pour le reflux dans la colonne de rectification.